# EUROPÄISCHE PATENTSCHRIFT

(11) **EP 1 343 538 B1**
(45) Veröffentlichungstag und Bekanntmachung des Hinweises auf die Patenterteilung: **01.03.2006**
(21) Anmeldenummer: 01989590.3
(22) Anmeldetag: 12.12.2001
(51) Int. Cl.: A61L 15/18

(54) **NANOSKALIGES ZNO IN HYGIENE-PRODUKTEN**
NANOMETRIC ZNO IN HYGIENE PRODUCTS
ZNO DE TAILLE NANOMETRIQUE DANS DES PRODUITS D'HYGIENE

(30) Priorität: 18.12.2000 DE 10063090
(43) Veröffentlichungstag der Anmeldung: 17.09.2003
(73) Patentinhaber: Henkel Kommanditgesellschaft auf Aktien, 40191 Düsseldorf (DE); Cognis Deutschland GmbH, 40589 Düsseldorf (DE)
(72) Erfinder: KROPF, Christian, 40271 Hilden (DE); HUNDEIKER, Claudia, 40549 Düsseldorf (DE); HELLER, Melita, 40591 Düsseldorf (DE); WILD, Christine, 40724 Hilden (DE)
(86) Internationale Anmeldenummer: PCT/EP2001/014562
(87) Internationale Veröffentlichungsnummer: WO 2002/049684

(56) Entgegenhaltungen:
- EP-A- 0 791 681
- WO-A-01/37888
- DE-A- 10 028 207
- US-B1- 6 337 362

## Beschreibung

Die vorliegende Erfindung betrifft das Gebiet von Hygiene-Produkten, insbesondere das Gebiet von Windeln für Babys und Erwachsene (Inkontinenz-Produkte), Slip-Einlagen und Tampons. Insbesondere betrifft die vorliegende Erfindung die Verwendung von nanoskaligem ZnO-Partikeln in solchen Hygiene-Produkten.

Hygiene-Produkte der oben beschriebenen Art werden zur Aufnahme von Urin, Fäzes, Blut und Schweiß, die der Körper ausgeschieden hat, eingesetzt. Da die oben genannten Ausscheidungen für ein feuchtes bis nasses Milieu sorgen, kann es in Folge dessen zu Reizungen und/oder Entzündungen der Haut wie Windeldermatitis kommen. Durch die Reibung des Hygiene-Produkts auf der Haut kann der Entzündungsprozeß zusätzlich beschleunigt werden.

Bekannt sind bereits Babywindeln, die auf der der Haut zugewandten Oberfläche (Vlies) eine Lotion zur Hautpflege enthalten (Procter & Gamble). Ebenfalls bekannt (WO99/59538) sind topische Zusammensetzungen, die ZnO mit großer Oberfläche (30 bis 100m²/g) und mit einer mittleren Partikelgröße von 0,1 bis 200 µm (im Durchmesser) enthalten. Diese Zusammensetzungen werden besonders empfohlen für die Absorption von Körperflüssigkeit, z.B. von Schweiß, Sebum (Talg), Urin und Wasser. Die Wirkung (z.B. bei der Behandlung von Akne oder Windelekzemen) des ZnO wird auf seine antibakterielle (antiseptische) und auch antiinflammatorische Wirksamkeit zurückgeführt. Letztere ist beispielsweise beschrieben bei Heinrich et al. in *Parfümerie und Kosmetik* 76, 88-91 (1995).

Die bekannten Produkte haben jedoch verschiedene ganz wesentliche Nachteile: Zunächst von nachteiliger Bedeutung ist die Tatsache, dass sich die hydrophilen ZnO-Partikel in hydrophobe Zusammensetzungen nur schlechte bis gar nicht einarbeiten lassen (es sei denn, man umschließt die ZnO-Partikel mit einem organischen Coating vollständig, wodurch aber wiederum seine antiseptische und anti-inflammatorische Wirkung behindert wird). Ein weiterer Nachteil liegt darin, dass die vergleichsweise großen Partikel bzw. Agglomerate auf der Haut für ein ungutes Gefühl verantwortlich sind. Ein weiterer Nachteil liegt in einem großen Partikelbedarf und auch in einer schlechten Stabilität in den Applikationssystemen aufgrund von Sedimentation der relativ großen Partikel. Schließlich liegt ein weiterer Nachteil der bekannten Produkte darin, dass eine erhöhte Gefahr von Hautirritationen durch Abrasion auf Grund großer Partikel/Agglomerate besteht.

Einige dieser Nachteile lassen sich nach dem heutigen Stand der Technik bereits vermeiden. Das sind all die oben genannten Nachteile, die mit der nicht genügend kleinen Partikelgröße im Zusammenhang stehen, denn die EP-A 0 791 681 beschreibt ZnO-Partikel einer durchschnitt-lichen Partikelgröße von nicht mehr als 100 nm, die zur Beschichtung von Substraten (wie synthetischen, natürlichen und anorganischen Fasern) geeignet sind. Die mit den ZnO-Partikel versehenen Substrate wirken einerseits antibakteriell und andererseits Gerüche unterdrückend.

Die Aufgabe, die sich die Erfinder gegenüber dem Stand der Technik zu lösen gestellt haben, besteht darin, Hygiene-Produkte im obigen Sinn bereit zu stellen, die ZnO als antibakteriellen und anti-inflammatorischen Wirkstoff enthalten, wobei dieses ZnO eine höhere Wirksamkeit besitzt und taktil nicht störend empfunden wird (das heißt, es soll gleichzeitig ein angenehmes Tragegefühl erreicht werden) und stabil in einer hautpflegenden Lipid/Wachs basierten Matrix verfügbar ist.

Dazu haben die Erfinder der vorliegenden Erfindung zahlreiche Formen von ZnO auf die gewünschten Eigenschaften getestet und festgestellt, dass verschiedene Formen von ZnO die gestellte Aufgabe zu lösen in der Lage sind, wenn sie an ihrer Oberfläche modifiziert sind und in möglichst wenig oder gar nicht agglomerierter Form vorliegen.

Ein Gegenstand der vorliegenden Erfindung betrifft somit die Verwendung von ZnO für die Herstellung von Hygiene-Produkten, wobei das ZnO in Form von an seiner Oberfläche chemisch oder physikalisch modifizierten Nanopartikeln vorliegt. Gemäß bevorzugter Ausführungsformen handelt es sich bei dem Hygiene-Produkt um eine Windel für Babys oder für Erwachsene, um eine Slip-Einlage oder um einen Tampon. Nach einer anderen bevorzugten Ausführungsform erfolgt die chemische oder physikalische Modifizierung der ZnO-Partikel-Oberfläche mit organischen Verbindungen, speziell mit (a) Carbonsäuren (Mono-, Di- und Polycarbonsäuren) bzw. deren Derivaten wie Anhydride, Halogenide und Ester (einschließlich der Laktone); insbesondere mit Stearinsäure, Palmitinsäure, Laurinsäure, Caprinsäure, Caprylsäure, Capronsäure, Ölsäure, Sorbinsäure, Linolsäure, Linolensäure, Ricinolsäure, Oxalsäure, Malonsäure, Bemsteinsäure, Glutarsäure, Adipinsäure, Zitronensäure, Äpfelsäure, Milchsäure, Weinsäure; mit (b) Aminosäuren, insbesondere mit den natürlich vorkommenden Aminosäuren (Gly, Ala, Val, Leu, Ile, Phe, Tyr, Trp, Pro, Hy-Pro, Ser, Asp, Glu, Asn, Gln, Arg, Lys, Thr, His, Cys, Met); mit (c) Hydroxy-Carbonsäuren und Zuckersäuren wie Glucarsäure, Gluconsäure, Glucuronsäure; mit (d) Polyglykolsäuren der allgemeinen Formel HOOC-CH₂-O-(CH₂-CH₂-O)ₙ-CH₂-COOH, wobei n Null oder eine ganze Zahl von 1 bis 100, vorzugsweise 1, 2, 3, 4, 5, 6, 7, 8, 9, 10, 11 oder 12 ist; mit (e) Ethercarbonsäuren der allgemeinen Formel R-(O-CH₂-CH₂)ₙ-O-CH₂-, COOH, wobei n Null oder eine ganze Zahl von 1 bis 100, vorzugsweise 1, 2, 3, 4, 5, 6, 7, 8, 9, 10, 11 oder 12 ist, und wobei R ein Alkyl, Alkenyl oder Alkinyl-Rest ist; vorzugsweise wobei R = C₆-, C₈-, C₁₀-, C₁₂-, C₁₄-, C₁₆-, C₁₈-Alkyl, -Alkenyl oder - Alkinyl; mit (f) Alkylhalogeniden; oder mit (g) Silanen des Typs (OR)₄₋ₙSiR'ₙ, wobei R ein Alkyl-Rest ist, vorzugsweise R = Methyl, Ethyl, Propyl, i-Propyl, Butyl, t-Butyl und R' ein organischer, insbesondere ein aliphatischer, Rest mit ggf. funktionellen Gruppen wie -OH, -COOH, Ester, Amin oder Epoxy ist, wobei vorzugsweise R' = C₆-, C₈-, C₁₀-, C₁₂-, C₁₄-, C₁₆-, C₁₈-Alkyl, Alkenyl oder -Alkinyl, Aminopropyl, N-Aminoethyl-3-aminopropyl, n- oder i-Propyl-N,N,N,-dimethyloctadecylammoniumchlorid, n- oder i-Propyl-N,N,N-trimethylammoniumchlorid, n- oder i-Propylbem-steinsäureanhydrid.

Ein weiterer Gegenstand der vorliegenden Erfindung betrifft ein Verfahren zur Herstellung von Hygiene-Produkten, wobei das ZnO in Form von an seiner Oberfläche chemisch oder physi-kalisch modifizierten Nanopartikeln auf die Oberfläche des Hygiene-Produkts aufgetragen wird.

Ein weiterer Gegenstand der vorliegenden Erfindung betrifft schließlich ein Hygiene-Produkt enthaltend ZnO, wobei dieses an seiner Oberfläche chemisch oder physikalisch modifiziert ist.

Die mittlere Primärteilchengröße der ZnO-Nanopartikei (Durchmesser) gemäß der vorliegenden Erfindung liegt im Bereich von 1 - 100 nm, bevorzugter Weise im Bereich von 1 - 50 nm bzw. 5 - 40 nm und 10 - 20 nm. Besonders bevorzugte Werte(bereiche) für die mittlere Partikelgröße liegen bei 5 bis 20 nm, bei 10 - 25 nm oder bei 15 - 35 nm.

Die spezifische Oberfläche der Partikel ist mindestens 10 m²/g, bevorzugt sind Werte von mindestens 40 m²/g bzw. mindestens 100 m²/g.

Dementsprechend betrifft die vorliegende Erfindung Hygiene-Produkte oder Teile davon (die mit der Haut in Berührung stehen, insbesondere Vlies-Materialien), die nanoskalige ZnO-Partikel (ZnO-Nanopartikel) enthalten und die aufgrund dieser Partikel antibakteriell (antiseptisch) und/oder entzündungshemmend wirken. Diese sogenannten ZnO-Nanopartikel (oder kurz Nanopartikel) sind bevorzugter Weise solche Modifikationen von ZnO, die als an der Oberfläche chemisch oder physikalisch modifizierte Nanopartikel vorliegen. Nach einer besonders bevorzugten Ausführungsform erfolgt die chemische oder physikalische Modifizierung der ZnO-Partikel mit einer Carbonsäure bzw. einem ihrer Derivate (wie Anhydrid, Halogenid oder Ester), mit einer Aminosäure, mit einer HydroxyCarbonsäure oder einer Zuckersäure, mit einer Polyglykolsäure der allgemeinen Formel HOOC-CH₂-O-(CH₂-CH₂-O)ₙ-CH₂-COOH, mit einer Ethercarbonsäure der allgemeinen Formel R-(O-CH₂-CH₂)ₙ-O-CH₂-COOH, mit einem Alkylhalogenide oder mit einem Silan des Typs (OR)₄₋ₙSiR'ₙ. Speziell bevorzugt als modifizierendes Agens sind Carbonsäuren, insbesondere Fettsäuren. Ganz speziell bevorzugt ist Stearinsäure.

Grundsätzlich weisen "ultrakleine" Teilchen (Nanopartikel) Eigenschaften auf, die sich grund-legend von denen größerer Teilchen unterscheiden. Sie streuen unter gewissen Umständen kein Licht, da sie wesentlich kleiner als die Wellenlänge des Lichtes sind. Sie können also trans-parente Formulierungen ergeben, wenn sie auf Primärpartikelgröße dispergiert sind. Sie besitzen eine sehr große spezifische Oberfläche (30 - 300 m²/g) und deshalb auch eine hohe Reaktivität.

Zur vollen erfindungsgemäßen Entfaltung ihrer Eigenschaften müssen die Nanopartikel kleiner als 100 nm sein. Bevorzugt werden Teilchengrößen zwischen 2 und 60 nm angestrebt. Ein weiteres wesentliches Kriterium für die erfindungsgemäße Qualität der Nanoteilchen ist eine enge Partikelgrößenverteilung, damit die Teilchen möglichst monodispers vorliegen. Mit anderen Worten ausgedrückt heißt dies, dass die Teilchen- bzw. Partikel-Agglomeration kontrolliert werden sollte, um übermäßige Agglomeration zu vermeiden.

Um das Potential der Nanopartikel erfindungsgemäß optimal nutzen zu können, benötigt man Herstellungsverfahren, die es erlauben, größere Mengen nanokristalliner Stoffe mit kontrollierter Teilchengröße und schmaler Teilchengrößenverteilung zu präparieren. Der apparative Aufwand muß dabei überschaubar sein, damit die Kosten niedrig gehalten werden können. Solche Verfahren sind im Stand der Technik bekannt, sollen nachfolgend aber dennoch kurz skizziert werden, um die vorliegende Erfindung besser zu verdeutlichen.

Zunächst müssen die nanoskaligen Partikel hergestellt werden, die anschließend weiter behandelt werden müssen, um die Teilchen-Agglomeration zu kontrollieren. Deshalb sollen nachfolgend jeweils zuerst solche Herstellungsverfahren und daran anschließend Behandlungs- bzw. Modifizierungsverfahren beschrieben werden, die die Agglomeration unterbinden. Die Nanopartikel werden erfindungsgemäß also in an ihrer Oberfläche chemisch oder physikalisch modifizierter Form für Hygiene-Produkte verwendet.

Im wesentlichen lassen sich die Herstellungsverfahren für Nanopartikel (ganz allgemein) auf Basis anorganischer Materialien (Oxide, Nitride, Metalle usw.) einteilen in Synthesen über flüssige Phasen (dazu zählen der Sol/Gel-Prozeß, die Fällungsreaktion und die Mikroemulsion) und Gasphasenverfahren.

### Flüssige Phase

Im **Sol/Gel-Prozeß** werden hydrolysierbare molekulare Ausgangsverbindungen (z.B. ZnCl₂ oder Zn(OPr)₂, wobei OPr OC₃H₇, d.h., n-Propoxy oder Isopropoxy, bedeutet) kontrolliert mit Wasser (ggf. unter Zugabe eines Katalysators) zur Reaktion gebracht (übersichtsartig beschrieben in EP-B 0 774 443, Seite 2, [0004] bis [0011], allerdings für TiO₂, was für ZnO aber analog Gültigkeit besitzt, sowie den dort genannten Literaturstellen). Die Hydrolyseprodukte kondensieren im Anschluß zu oxidischen Nanoteilchen. Diese Teilchen besitzen eine extrem große und reaktive Oberfläche, so dass an der Oberfläche der Teilchen befindliche OH-Gruppen miteinander reagieren (Kondensation) und so die Agglomeration einleiten. Diese Agglomeration kann durch während des Sol/Gel-Prozesses anwesende Schutzkolloide oder Tenside verhindert werden: Die polaren Gruppen belegen die Teilchenoberfläche und sorgen sowohl für eine sterische als auch für eine elektrostatische Abstoßung der Teilchen.

Eine weitere Methode zur Verhinderung von Aggregaten ist die Oberflächenmodifizierung des Materials mit Carbonsäuren und Alkoxysilanen. Bei dieser Methode wird die Reaktivität der Partikel für deren (partielle) Desaktivierung ausgenutzt: Die freien OH-Gruppen werden entweder verestert (Carbonsäuren) oder silaniert. In beiden Fällen kommt es zur Ausbildung kovalenter Bindungen zwischen den Partikeloberflächen und der oberflächenwirksamen Substanz. Länge und Funktionalität des organischen Restes bestimmen im wesentlichen die Dispergierbarkeit des Materials in verschiedenen Medien.

Bei der **Fällungsreaktion** werden gelöste Ionen durch Zugabe eines geeigneten Fällungsreagenzes (häufig durch Verschieben des pH-Wertes) gefällt (für TiO₂ wiederum beschrieben in EP-B 0 774 443, Seiten 3 bis 6, [0019] bis [0065]). Durch thermische Nachbehandlung lassen sich kristalline Pulver erhalten, die normalerweise jedoch Agglomerate enthalten. Im allgemeinen kann in gewissem Umfang die mittlere Teilchengröße, die Teilchengrößenverteilung, der Kristallirütätsgrad, unter Umständen sogar die Kristallstruktur und der Dispersionsgrad, über die Reaktionskinetik beeinflußt werden.

Setzt man beim Fällungsprozeß oberflächenaktive Substanzen wie Polycarbonsäuren, Tenside oder Polyalkohole zu, belegen diese die Oberflächen der wachsenden Keime und verhindern so ein unkontrolliertes Weiterwachsen der Partikel. Die Oberflächenbelegung unterstützt zudem die spätere Redispergierbarkeit der isolierten Pulver. Diese Variante der Fällungsreaktion wird aus diesem Grund zur Herstellung nanoskaliger Pulver bevorzugt und eignet sich besonders für die Herstellung von erfindungsgemäßem ZnO.

Bei **Mikroemulsionen** (ME) nutzt man die wäßrigen Phasen von w/o-Emulsionen als Reaktionsräume für die Darstellung nanoskaliger Materialien aus. Alle Reaktionen, die in wäßrigen Medien zur Darstellung nanoskaliger Materialien dienen, lassen sich also im Prinzip auch in Mikroemulsionen durchführen. Das gilt besonders für die Fällungsreaktionen und den Sol/Gel-Prozeß. Das Wachstum der Teilchen wird hierbei begrenzt durch die Größe des Reaktionsraumes der nmgroßen Tröpfchen. Eine Reihe von Übersichtsartikeln geben einen Überblick über ME als Reaktionsmedien zur Darstellung nanoskaliger Materialien [z.B. Chhabra et al., *Tenside, Surfactants, Deterg.* 34, 156-168 (1997); Eastoe et al., *Curr. Opin. Colloid Interface Sci*. 1, 800-805 (1996); Schwuger et al., *Chem. Rev.* 95, 849-864 (1995); Lopez-Quintela et al., *J. Colloid Interface Sci*. 158, 446-451 (1993)].

Weitere Behandlungs- bzw. Modifizierungsverfahren inklusive Oberflächenmodifikatoren, die sich alle für die erfindungsgemäße Verwendung eignen, sind beschrieben in der WO96/34829, WO97/38058, WO98/51747, EP-B 0 636 111 und DE-A 43 36 694.

### Gasphase

In den vergangenen 10 Jahren wurden zahlreiche Gasphasenprozesse neu bzw. weiterentwickelt, so dass ausreichend Prozesse zur Verfügung stehen (z.B. Kruis et al., *J. Aerosol. Sci*. 29, 511 (1998)). Diese Verfahren in der Gasphase führen wegen des hohen Drucks (bei gleichzeitig gro-ßer Produktionsrate) zu starker Agglomeration der Nanoteilchen schon im Herstellungsprozeß, d.h. die reaktiven Teilchen lagern sich durch Sintervorgänge zu größeren Agglomeraten zusammen, so dass es erfindungsgemäß erforderlich ist, ein Verfahren zur Kontrolle der Agglomeration, das heisst ein Verfahren zur Modifizierung der Nanopartikel, anzuschließen.

Um die Qualität der Nanopartikel, das heißt unter anderem, ihre mittlere Teilchenverteilung, beurteilen zu können, stehen verschiedene Methoden zur Verfügung, von denen die wichtigsten im Folgenden kurz erläutert werden sollen.

Die Methode der Transmissions-Elektronen-Mikroskopie (TEM) erfordert neben einem hohen apparativen Aufwand auch viel Fingerspitzengefühl vom Operator und ist deshalb als Standard-Labormethode ungeeignet. Die Röntgenbeugung macht sich die Auswertung der Breite von Röntgenbeugungsreflexen zunutze und ergibt Hinweise auf die Größe der im Material vorhandenen Primärpartikel. Die Linienbreite ergibt sich aus der instrumentellen Breite (Auf-lösung), der Verbreiterung aufgrund kleiner Teilchengrößen und der Verbreiterung aufgrund von Mikrospannungen. Unter der Annahme, dass die Verbreiterung der Reflexe hauptsächlich durch kleine, kugelförmige Teilchen hervorgerufen wird, erhält man durch Anwendung der Scherrer-Gleichung die Volumen gemittelte Größe der untersuchten Kristallite.

Zur Größenbestimmung kolloidaler Teilchen bietet sich ferner die dynamische Lichtstreuung an, die mittlerweile zur Standardmethode herangereift ist (Powder and Bulk Engineering, Febr. 1995, 37-45). Der Vorteil dieser Methode liegt in der einfachen und schnellen Handhabung. Nachteilig ist hingegen, dass Viskosität des Dispergiermediums und Brechungsindex des Partikels bekannt sein müssen.

Die Methoden der BET-Isotherme und OH-Gruppen-Dichte können als Routinemethoden zur weiteren Charakterisierung des Materials eingesetzt werden.

Durch Aufnahme der BET-Isotherme erhält man die spezifische Oberfläche des Materials. Im Falle von geringfügig agglomeriertern Pulver sollte also die gemessene BET-Oberfiläche von der für isolierte Teilchen berechneten nur unwesentlich abweichen. Größere Differenzen geben somit einen direkten Hinweis auf größere und dichtere Agglomerate/Aggregate (Versinterung), obwohl die Primärteilchen gemäß Röntgenbeugung sehr klein sein können.

Die Dichtebestimmung der Hydroxylgruppen auf den Oberflächen der Pulver gibt einen wichtigen Hinweis auf die Reaktivität und die Funktionalisierbarkeit: Eine geringe Dichte bedeutet, dass das Material während der Synthese sehr hohen Temperaturen ausgesetzt war und zumindest teilweise "totgebrannt" ist. Eine hohe Hydroxylgruppendichte erleichtert die Funktionalisierung und Stabilisierung der Teilchen und wird deshalb bevorzugt.

Zur Ermittlung der OH-Gruppendichte wird das Pulver mit Thionylchlorid umgesetzt (Austausch OH → CI) und anschließend quantitativ hydrolysiert (Freisetzung der Chloridionen). Die Titration der Chloridionen ergibt bei Kenntnis der spezifischen Oberfläche den Wert für die Hydroxylgruppendichte.

Die Verwendung von an ihrer Oberfläche chemisch oder physikalisch modifizierten ZnO-Nanopartikeln für die erfindungsgemäßen Hygieneprodukte ist beispielsweise im Gegensatz zu herkömmlichem (unmodifiziertem) ZnO mit einer mittleren Partikelgröße im Mikrometer-Bereich (bekannt z.B. aus WO99/59538) aus verschiedenen Gründen eindeutig zu bevorzugen. Erstens läßt sich das nanoskalige Material leichter formulieren (ohne dass es zu unnötig starker Sedimentation der Partikel kommt), da die Modifizierung die hydrophile Eigenschaft der ZnO-Partikel reduziert und damit die Formulierung mit (hydrophoben) Cremes erleichtert. Weiterhin ist die Wirksamkeit des ZnO in Folge seiner vergrößerten spezifischen Oberfläche bei gleicher Menge an eingesetzten Nanopartikeln höher (was aber nichts mit der Modifizierung zu tun hat). Schließlich führt die geringe Partikelgröße auch zu einer verbesserten Sensorik (Taktilität) auf der Haut: es wird kein kömiges Gefühl empfunden wie bei den herkömmlichen ZnO-Partikeln. Darüber hinaus kann die abrasive Eigenschaft der Partikel bei geringerer Partikelgröße geringer sein, und somit wird die Beanspruchung (mechanische Schädigung) der Haut mit abnehmender Partikelgröße reduziert.

Die erfindungsgemäß relevanten Eigenschaften des ZnO sind zum einen seine antibakterielle Wirkung, zum anderen die Haut beruhigende (antiinflammatorische) Wirkung. Beide hängen davon ab, dass die Oberfläche der ZnO-Partikel in Folge der Modifizierung keine Beschichtung in dem Sinne ist, dass die nanoskaligen Partikel völlig bedeckt sind, sondern dass Zn-lonen durch die modifizierte Oberfläche an die Umgebung abgegeben werden können. Konkreter bedeutet Modifizierung die Belegung der Partikel-Oberfläche mit organischen Verbindungen, die über chemische Bindungen oder physikalische Kräfte mit der Oberfläche der Partikel wechselwirken.
Oberflächenmodifikatoren, die erfindungsgemäß Verwendung finden können, sind z.B. alle als solche in den Druckschriften WO96/34829 (Seite 8, Zeile 20, bis Seite 9, Zeile 7), WO97/38058 (Seite 5, Zeile 28, bis Seite 6, Zeile 17), WO98/51747 (Seite 5, 2. Absatz, bis Seite 8, 1. Absatz), EP-B 0 636 111 (Spalte 3, Zeile 38, bis Spalte 4, Zeile 56) und DE-A 43 36 694 (Spalte 6, Zeilen 1/63) genannten Verbindungen. Zur Modifizierung bevorzugte Verbindungen sind insbesondere
(a) Carbonsäuren (Mono-, Di- und Polycarbonsäuren) bzw. deren Derivate wie Anhydride, Halogenide und Ester (einschließlich der Laktone); insbesondere Stearinsäure, Palmitinsäure, Laurinsäure, Caprinsäure, Caprylsäure, Capronsäure, Ölsäure, Sorbinsäure, Linolsäure, Linolensäure, Ricinolsäure, Oxalsäure, Malonsäure, Bemsteinsäure, Glutarsäure, Adipinsäure, Zitronensäure, Äpfelsäure, Milchsäure, Weinsäure;
(b) Aminosäuren, insbesondere die natürlich vorkommenden Aminosäuren (Gly, Ala, Val, Leu, Ile, Phe, Tyr, Trp, Pro, Hy-Pro, Ser, Asp, Glu, Asn, Gln, Arg, Lys, Thr, His, Cys, Met);
(c) Hydroxy-Carbonsäuren und Zuckersäuren wie Glucarsäure, Gluconsäure, Glucuronsäure;
(d) Polyglykolsäuren der allgemeinen Formel HOOC-CH₂-O-(CH₂-CH₂-O)ₙ-CH₂₋COOH, wobei n vorzugsweise 1, 2, 3, 4, 5, 6, 7, 8, 9, 10, 11 oder 12 ist;
(e) Ethercarbonsäuren der allgemeinen Formel R-(O-CH₂-CH₂)ₙ-O-CH₂-COOH, wobei vorzugsweise R = C₆-, C₈-, C₁₀-, C₁₂-, C₁₄-, C₁₆-, C₁₈-Alkyl, -Alkenyl oder -Alkinyl;
(f) Alkylhalogenide;
(g) Silane des Typs (OR)₄₋ₙSiR'ₙ, wobei vorzugsweise R = Methyl, Ethyl, Propyl, i-Propyl, Butyl, t-Butyl und R' ein organischer, insbesondere ein aliphatischer, Rest mit funktionellen Gruppen wie -OH, -COOH, Ester, Amin oder Epoxy ist, wobei vorzugsweise R' = C₆-, C₈-, C₁₀-, C₁₂-, C₁₄-, C₁₆-, C₁₈-Alkyl, -Alkenyl oder -Alkinyl, Aminopropyl, N-Aminoethyl-3-aminopropyl, n- oder i-Propyl-N,N,N,-dimethyloctadecylammoniumchlorid, n- oder i-Propyl-N,N,N-trimethylammoniumchlorid, n- oder i-Propylbemsteinsäureanhydrid.

Andere Modifikatoren sind Tenside wie Fettalkohol (FA)-Derivate und Alkylpolyglucoside (APGs), Polymere wie Polyethylenglykole, Polypropylenglykole, Polyvinylalkohole, Polyvinylpyrrolidon, Polyvinylbutyrole oder Polyasparaginsäure, oder Schutzkolloide (z.B. Gelatine, Stärke, Dextrin, Dextran, Pektin, Casein, gummi arabicum) sowie deren Derivate oder Mischungen von diesen.

Die Modifizierung wird, wie auch in den oben erwähnten Druckschriften beschrieben, je nach Löslichkeit der zur Modifizierung verwendeten Substanz in Wasser, Alkohol (Ethanol, n-Propanol, i-Propanol, Propylenglykol), Ether (Tetrahydrofuran, Diethylether) oder einem aprotischen Lösungsmittel (LM) wie Hexan, Cyclohexan, Heptan, i-Oktan, Toluol durchgeführt.

Das zu modifizierende Pulver wird im LM dispergiert und ggf. durch Kochen am Wasserabscheider von Wasserresten befreit. Anschließend wird das Modifizierungsreagenz zugegeben und unter Rückfluß erhitzt auf eine Temperatur zwischen RT und dem Siedepunkt des LM (bei Normaldruck). Dabei wird entstehendes Wasser ggf. am Wasserabscheider abgetrennt. Anschließend wird das Pulver z.B. mittels Filtration oder Zentrifugation von der Suspension abgetrennt, gewaschen und optional getrocknet (Trockenschrank, Gefriertrocknung).

Die Applikation der an ihrer Oberfläche chemisch oder physikalisch modifizierten Nanopartikel auf das Hygiene-Produkt erfolgt nach Verfahren, die aus dem Stand der Technik bekannt sind, und kann z.B. durch Tränken (Foulard), Rollenapplikation oder Besprühen des Hygiene-Produkts mit einer Lösung/Suspension der Nanopartikel enthaltenden Avivage und anschließendes Trocknen erfolgen.

Die Nanopartikel können sowohl in wasserfreien als auch in wässrigen Systemen suspendiert bzw. gelöst werden. Sowohl die wasserfreien als auch die wässrigen Systeme können einerseits aus hydrophoben, andererseits aber auch aus hydrophilen Komponenten zusammengesetzt sein, um den Hygiene-Produkten ein für die unterschiedlichen Anwendungsbereiche notwendiges hydrophiles oder hydrophobes Verhalten zu verleihen. Soll das Vlies Flüssigkeit absorbieren, ist er hydrophil ausgerüstet; soll er dagegen Flüssigkeit abweisen, muß er hydrophob sein. So ist der mittlere Teil eines Topsheets (oberstes Vlies einer Windel) hydrophil, um die Flüssigkeit aufnehmen und an die tieferen Schichten weiterleiten zu können. Der äußere Teil des Topsheets ist hingegen hydrophob, um eine Leckage zu verhindern. Für beide Bereiche aber ist eine antibakterielle und entzündungshemmende Ausrüstung erwünscht.

Der Nanopartikel-Gehalt einer solchen (oben genannten) Avivage liegt im Bereich von 0,1 bis 50 Gew.%, bevorzugter Weise im Bereich von 0,5 bis 30 Gew.%, besonders bevorzugter Weise im Bereich von 1 bis 10 Gew.%.

Eine weitere Möglichkeit, die Nanopartikel auf das Hygiene-Produkt aufzubringen, besteht in der Einarbeitung der Nanopartikel in eine (hautpflegende hydrophobe) Lotion, bevorzugter Weise auf Wachs-Basis, die auf das Vlies-Material/die Gewebelage aufgetragen wird. Die Applikation der Wachse kann während der Herstellung des Vlies oder bei der Herstellung des gebrauchsfertigen Hygiene-Produkts (z. B. Windel) erfolgen.

Diese Ausführungsform ist besonders bevorzugt, da der Gehalt der Nanopartikel in der Lotion geringer ist als bei der Avivage (da die Auftragsmenge an Lotion höher ist) und liegt im Bereich von 0,1 bis 10 Gew.%, bevorzugter Weise im Bereich von 0,1 bis 8 Gew.%.

### Beispiele

### Beispiel 1: Modifizierung von nanoskaligem (nano-)ZnO mit Stearinsäure

60 g nano-ZnO wurden in 250 ml n-Octan dispergiert und am Wasserabscheider von anhaftendem Wasser befreit (ca. 1 ml). Danach wurden 10,7 g Stearinsäure (98%ig) zugegeben und das Gemisch 5 h unter Rückfluß gekocht. Dabei wurden weitere 0,5 ml Wasser abgeschieden. Das anschließend erhaltene, an der Oberfläche chemisch oder physikalisch modifizierte nanoskalige ZnO-Pulver wurde mittels Zentrifugation abgetrennt, mit n-Octan gewaschen und zunächst an Luft, dann ca. 8 h bei 50°C im Umlufttrockenschrank getrocknet

### Beispiel 2: Modifizierung von nanoskaligem ZnO mit Ethercarbonsäure

Da die Ethercarbonsäure R-(O-CH₂-CH₂)_{2,5}-O-CH₂-COOH (R = C₁₂₋₁₄) herstellbedingt Wasser enthält, wurden zunächst 2,7 g Akypo RLM 25 (92%ig, Handelsname der Fa. Kao) in 200 ml n-Hexan gelöst und am Wasserabscheider gekocht, bis das Wasser vollständig abgetrennt war (es handelt sich bei der obigen Formel um die Beschreibung des mittleren Polymerisationsgrades der EO-Gruppen). Anschließend wurden 92 g nano-ZnO in diese Lösung eindispergiert und 4 h am Rückfluß gekocht. Entstehendes Wasser (2,8 ml) wurde wie zuvor abgeschieden. Anschließend wurde das modifizierte Pulver durch Filtration abgetrennt, mit n-Hexan gewaschen und 4-5 h bei 50°C im Umlufttrockenschrank getrocknet.

### Beispiel 3: Untersuchungen am humanen dreidimensionalen Hautmodell

Es wurde eine PIT (Phasen-Inversions-Temperatur)-Creme mit herkömmlichem ZnO bzw. mit nanoskaligem ZnO, das mit Stearinsäure gecoated war, hergestellt. Diese Cremes wurden am humanen dreidimensionalen Hautmodell (Fa. Matek Corp., MA Ashland, USA) bzgl. ihres Einflusses auf die Vitalität bzw. auf die Ausschüttung von Entzündungsmediatoren (Interleukin-1α, Prostaglandin E2) hin untersucht.

Auf vier Hautmodelle wurde demineralisiertes Wasser (Aqua demin.) appliziert. Alle anderen Hautmodelle wurden mit 80µl einer 0,16%igen Na-Laurylsulfat (SDS)-Lösung für eine Stunde inkubiert (37°C, 5% CO₂, 90% rel. Luftfeuchtigkeit). Anschließend wurden die Hautmodelle mit Phosphatpuffer gewaschen und dann PIT-Creme 1 (mit herkömmlichem ZnO) und PIT-Creme 2 (mit Stearinsäuregecoatetem nanoskaligem ZnO) appliziert. Es wurden jeweils Vierfach-Bestimmungen durchgeführt. Als Kontrolle wurde auf jeweils vier Hautmodelle Cortison-Creme (SDS/Aqua demin.) und auf vier Hautmodelle Aqua demin. (Aqua demin./Aqua demin.) appliziert.

Nach einer 24-stündigen Inkubation (37°C, 5% CO₂, 90% rel. Luftfeuchtigkeit) wurden die Hautmodelle wiederum mit Phosphatpuffer gewaschen. Im Anschluß wurde die Haut mittels MTT-Assay (Methylthiazoltetrazolium) auf ihre Vitalität hin untersucht und im Medium die Ausschüttung der Entzündungsmediatoren Interleukin 1-α und Prostaglandin E2 bestimmt.

**Tabelle 1: PIT-Zinkoxid - Cremes für Versuche an humanen Hautmodellen**

| **Inhaltsstoffe** | **Creme 1** | **Creme 2** |
|---|---|---|
| | **(Gew.%)** | **(Gew.%)** |
| **INCI** | 1 | 2 |
| Dicaprylyl Ether | 12 | 12 |
| Decyl Oleat | 5 | 5 |
| Cetearyl Alcohol | 4 | 4 |
| Hydrierte Palm-Glyceride | 2 | 2 |
| Ceteareth 20 | 2,5 | 2,5 |
| Herkömmliches ZnO (nicht nanoisiert, in Wasser vordispergiert) | 3 | - |
| Nanoisiertes ZnO (modifiziert mit Stearinsäure, in Dicaprylylether vordispergiert) | - | 3 |
| Glycerin | 5 | 5 |
| Wasser | 66 | 66 |
| Phenoxyethanol, Methylparaben, Ethylparaben, Propylparaben, Butylparaben | 0,5 | 0,5 |
| Die Emulsionen wurden in einem 2-Schritt-Verfahren hergestellt. Das ZnO wurde entweder in Dicaprylylether (2) oder in Wasser (1) vordispergiert. | | |

Die Bestimmung der Entzündungsmediatoren erfolgte mittels ELISA-Assay (Enzyme Linked Immuno Sorbent Assay).

### Ergebnis:

Die Behandlung der Hautmodelle mit Na-Laurylsulfat-Lösung und im Anschluß daran mit Aqua demin. (SDS/Aqua demin.) führte zu einer Reduktion der Vitalität der Hautmodelle sowie zu einer verstärkten Ausschüttung von Interleukin-1α und Prostaglandin E2. Bei Behandlung der Hautmodelle mit Cortison-Creme nach der Inkubation mit Na-Laurylsulfat-Lösung wurde die Ausschüttung von Prostaglandin E2 deutlich, die von Interleukin-1α nur unwesentlich vermindert. Eine Behandlung mit PIT-Creme 1 bzw. mit PIT-Creme 2 führte zu einer leichten Verminderung der Vitalität. Eine Reduktion des Entzündungsmediators Interleukin-1α wurde jedoch nur nach Behandlung mit PIT-Creme 2 erreicht, die das nanoskalige, mit Stearinsäure gecoatete ZnO enthielt, nicht mit PIT-Creme 1. Tendenziell verminderte Creme 2 auch die Ausschüttung von Prostaglandin E2 im Vergleich zu Creme 1.

**Tabelle 2: Vitalität der Hautmodelle Vitalität (MTT-Test)**

| | Vitalität [% bezogen auf Aqua demin./Aqua demin.] | | |
|---|---|---|---|
| Ansätze | Einzelwerte | Mittelwert | Standardabweichung |
| Aqua demin / | 106 | **100** | 6 |
| Aqua demin | 95 | | |
| | 96 | | |
| | 103 | | |
| SDS / | 85 | **71** | **11** |
| Aqua demin. | 73 | | |
| | 58 | | |
| | 68 | | |
| SDS / | 105 | **105** | 11 |
| Cortison | 111 | | |
| | 89 | | |
| | 113 | | |
| Creme 1 | 94,291 | **90** | 13 |
| | 80,149 | | |
| | 79,007 | | |
| | 105,621 | | |
| Creme 2 | 71,366 | **83** | 10 |
| | 80,676 | | |
| | 84,892 | | |
| | 95,784 | | |

**Tabelle 3: Ausschüttung des Entzündungsmediators Interleukin-1α Interleukin 1-α**

| | Interleukin-1α [pg] | | |
|---|---|---|---|
| Ansätze | Einzeiwerte | Mittelwert | Standardabweichung |
| Aqua demin / | 13,922 | **23** | |
| Aqua demin | 10,099 | | |
| | 24,031 | | |
| | 41,977 | | |
| SDS / Aqua demin. | 143,957 | **140** | 10 |
| | 126,615 | | |
| | 149,783 | | |
| | 141,052 | | |
| SDS / Cortison | 108, 868 | **136** | 18 |
| | 147,839 | | |
| | 142,665 | | |
| | 142,988 | | |
| Creme 1 | 158,563 | **134** | 20 |
| | 133,975 | | |
| | 108,553 | | |
| | 133,654 | | |
| Creme 2 | 66,087 | **69** | 7 |
| | 72,112 | | |
| | 77,271 | | |
| | 62,198 | | |

**Tabelle 4: Ausschüttung des Entzündungsmediators Prostaglandin E2 Prostaglandin E2**

| | Prostaglandin E2 [pg] | | |
|---|---|---|---|
| Ansätze | Einzelwerte | Mittelwert | Standardabweichung |
| Aqua demin / | 3634,930 | **3999** | 615 |
| Aqua demin | 3434,488 | | |
| | 4108,748 | | |
| | 4819,096 | | |
| SDS / | 5721,413 | **8107** | 1690 |
| Aqua demin. | 8470,543 | | |
| | 9711,644 | | |
| | 8524,521 | | |
| SDS / | 712,733 | **869** | 323 |
| Cortison | 696,211 | | |
| | 1352,960 | | |
| | 712,733 | | |
| Creme 1 | 23194,855 | **14611** | 6252 |
| | 8363,998 | | |
| | 14395,119 | | |
| | 12488,945 | | |
| Creme 2 | 4942,001 | **9518** | 4662 |
| | 15677,425 | | |
| | 7134,079 | | |
| | 10320,418 | | |

## Patentansprüche (Patentansprüche für folgende(n) Vertragsstaat(en): AT, BE, CH, CY, DK, ES, FI, FR, GB, GR, IE, IT, LI, LU, MC, NL, PT, SE, TR)

1. Verwendung von ZnO für die Herstellung von Hygiene-Produkten, wobei das ZnO in Form von an seiner Oberfläche chemisch oder physikalisch modifizierten Nanopartikeln vorliegt, und wobei unter Modifizierung die Belegung der Partikel-Oberfläche mit organischen Verbindungen zu verstehen ist, die über chemische Bindungen oder physikalische Kräfte mit der Oberfläche der Partikel wechselwirken.

2. Verwendung nach Anspruch 1, wobei die Hygiene-Produkte ausgewählt sind aus Windeln für Babys und Erwachsene, Slip-Einlagen und Tampons.

3. Verwendung nach Anspruch 1 oder 2, wobei das ZnO antibakteriell (antiseptisch) und entzündungshemmend wirkt.

4. Verwendung nach einem der vorhergehenden Ansprüche, wobei das ZnO in Form von an seiner Oberfläche chemisch oder physikalisch modifizierten Nanopartikeln vorliegt, deren mittlere Primärpartikelgröße (Durchmesser) im Bereich von 1 - 100 nm, bevorzugter Weise im Bereich von 1 - 50 nm bzw. 5 - 40 nm und 10 - 20 nm, besonders bevorzugter Weise bei 5 bis 20 nm, bei 10 - 25 nm oder bei 15 - 35 nm, liegt.

5. Verwendung nach einem der vorhergehenden Ansprüche, wobei das ZnO in Form der an seiner Oberfläche chemisch oder physikalisch modifizierten Nanopartikel eine spezifische Oberfläche von mindestens 10 m²/g, bevorzugter Weise von mindestens 40 m²/g, und besonders bevorzugter Weise von mindestens 100 m²/g aufweisen.

6. Verwendung nach einem der vorhergehenden Ansprüche, wobei die chemische oder physikalische Modifizierung der Partikel-Oberfläche mit (a) Carbonsäuren (Mono-, Di- und Polycarbonsäuren) bzw. deren Derivaten wie Anhydride, Halogenide und Ester (einschließlich der Laktone); insbesondere mit Stearinsäure, Palmitinsäure, Laurinsäure, Caprinsäure, Caprylsäure, Capronsäure, Ölsäure, Sorbinsäure, Linolsäure, Linolensäure, Ricinolsäure, Oxalsäure, Malonsäure, Bernsteinsäure, Glutarsäure, Adipinsäure, Zitronensäure, Äpfelsäure, Milchsäure, Weinsäure; mit (b) Aminosäuren, insbesondere mit den natürlich vorkommenden Aminosäuren (Gly, Ala, Val, Leu, Ile, Phe, Tyr, Trp, Pro, Hy-Pro, Ser, Asp, Glu, Asn, Gln, Arg, Lys, Thr, His, Cys, Met); mit (c) Hydroxy-Carbonsäuren und Zuckersäuren wie Glucarsäure, Gluconsäure, Glucuronsäure; mit (d) Polyglykolsäuren der allgemeinen Formel HOOC-CH₂₋O-(CH₂-CH₂-O)ₙ-CH₂-COOH, wobei n 1, 2, 3, 4, 5, 6, 7, 8, 9, 10, 11 oder 12 ist; mit (e) Ethercarbonsäuren der allgemeinen Formel R-(O-CH₂-CH₂)ₙ-O-CH₂-COOH, wobei n 1, 2, 3, 4, 5, 6, 7, 8, 9, 10, 11 oder 12 und wobei R = C₆-, C₈-, C₁₀-, C₁₂-, C₁₄-, C₁₆-, C₁₈-Alkyl, -Alkenyl oder -Alkinyl; mit (f) Alkylhalogeniden; oder mit (g) Silanen des Typs (OR)₄₋ₙSiR'ₙ, wobei R = Methyl, Ethyl, Propyl, i-Propyl, Butyl, t-Butyl und R' = C₆-, C₈-, C₁₀-, C₁₂-, C₁₄-, C₁₆-, C₁₈-Alkyl, -Alkenyl oder -Alkinyl, Aminopropyl, N-Aminoethyl-3-aminopropyl, n- oder i-Propyl-N,N,N,-dimethyloctadecylammoniumchlorid, n-oder i-Propyl-N,N,N-trimethylammoniumchlorid, n- oder i-Propylbemsteinsäureanhydrid, erfolgt.

7. Verwendung nach Anspruch 6, wobei die Modifizierung mit Stearinsäure erfolgt.

8. Hygiene-Produkt enthaltend ZnO, wie es in einem der vorhergehenden Ansprüche definiert ist.

9. Das Hygiene-Produkt nach Anspruch 8, wobei das Hygiene-Produkte eine Windel für Babys oder für Erwachsene, eine Slip-Einlage oder ein Tampon ist.

10. Verfahren zur Herstellung eines Hygiene-Produkts, das definiert ist wie in Anspruch 8 oder 9, **dadurch gekennzeichnet, dass** an der Oberfläche chemisch oder physikalisch modifizierte ZnO-Nanopartikel, wie sie in einem der Ansprüche 1 bis 7 definiert sind, auf die Oberfläche des Hygiene-Produkts aufgetragen werden.

11. Das Verfahren nach Anspruch 10, **dadurch gekennzeichnet, dass** das Auftragen des ZnO auf das Hygiene-Produkt durch Tränken (Foulard), Rollenapplikation oder Besprühen des Hygiene-Produkts mit einer Lösung/Suspension der die ZnO-Nanopartikel enthaltenden Avivage und anschließendes Trocknen erfolgt.

## Patentansprüche (Patentansprüche für folgende(n) Vertragsstaat(en): DE)

1. Verwendung von ZnO für die Herstellung von Hygiene-Produkten, welche ausgewählt sind aus Windeln für Babys und Erwachsene, Slip-Einlagen und Tampons, wobei das ZnO in Form von an seiner Oberfläche chemisch oder physikalisch modifizierten Nanopartikeln vorliegt, und wobei unter Modifizierung die Belegung der Partikel-Oberfläche mit organischen Verbindungen zu verstehen ist, die über chemische Bindungen oder physikalische Kräfte mit der Oberfläche der Partikel wechselwirken.

2. Verwendung nach Anspruch 1, wobei das ZnO antibakteriell (antiseptisch) und entzündungshemmend wirkt.

3. Verwendung nach einem der vorhergehenden Ansprüche, wobei das ZnO in Form von an seiner Oberfläche chemisch oder physikalisch modifizierten Nanopartikeln vorliegt, deren mittlere Primärpartikelgröße (Durchmesser) im Bereich von 1 - 100 nm, bevorzugter Weise im Bereich von 1 - 50 nm bzw. 5 - 40 nm und 10 - 20 nm, besonders bevorzugter Weise bei 5 bis 20 nm, bei 10 - 25 nm oder bei 15 - 35 nm, liegt.

4. Verwendung nach einem der vorhergehenden Ansprüche, wobei das ZnO in Form der an seiner Oberfläche chemisch oder physikalisch modifizierten Nanopartikel eine spezifische Oberfläche von mindestens 10 m²/g, bevorzugter Weise von mindestens 40 m²/g, und besonders bevorzugter Weise von mindestens 100 m²/g aufweisen.

5. Verwendung nach einem der vorhergehenden Ansprüche, wobei die chemische oder physikalische Modifizierung der Partikel-Oberfläche mit (a) Carbonsäuren (Mono-, Di- und Polycarbonsäuren) bzw. deren Derivaten wie Anhydride, Halogenide und Ester (einschließlich der Laktone); insbesondere mit Stearinsäure, Palmitinsäure, Laurinsäure, Caprinsäure, Caprylsäure, Capronsäure, Ölsäure, Sorbinsäure, Linolsäure, Linolensäure, Ricinolsäure, Oxalsäure, Malonsäure, Bernsteinsäure, Glutarsäure, Adipinsäure, Zitronensäure, Äpfelsäure, Milchsäure, Weinsäure; mit (b) Aminosäuren, insbesondere mit den natürlich vorkommenden Aminosäuren (Gly, Ala, Val, Leu, Ile, Phe, Tyr, Trp, Pro, Hy-Pro, Ser, Asp, Glu, Asn, Gln, Arg, Lys, Thr, His, Cys, Met); mit (c) Hydroxy-Carbonsäuren und Zuckersäuren wie Glucarsäure, Gluconsäure, Glucuronsäure; mit (d) Polyglykolsäuren der allgemeinen Formel HOOC-CH₂₋O-(CH₂-CH₂-O)ₙ-CH₂-COOH, wobei n 1, 2, 3, 4, 5, 6, 7, 8, 9, 10, 11 oder 12 ist; mit (e) Ethercarbonsäuren der allgemeinen Formel R-(O-CH₂-CH₂)ₙ-O-CH₂-COOH, wobei n 1, 2, 3, 4, 5, 6, 7, 8, 9, 10, 11 oder 12 und wobei R = C₆-, C₈-, C₁₀-, C₁₂-, C₁₄-, C₁₆-, C₁₈-Alkyl, -Alkenyl oder -Alkinyl; mit (f) Alkylhalogeniden; oder mit (g) Silanen des Typs (OR)₄₋ₙSiR'ₙ, wobei R = Methyl, Ethyl, Propyl, i-Propyl, Butyl, t-Butyl und R' = C₆-, C₈-, C₁₀-, C₁₂-, C₁₄-, C₁₆-, C₁₈-Alkyl, -Alkenyl oder -Alkinyl, Aminopropyl, N-Aminoethyl-3-aminopropyl, n- oder i-Propyl-N,N,N,-dimethyloctadecylammoniumchlorid, n-oder i-Propyl-N,N,N-trimethylammoniumchlorid, n- oder i-Propylbemsteinsäureanhydrid, erfolgt.

6. Verwendung nach Anspruch 5, wobei die Modifizierung mit Stearinsäure erfolgt.

7. Hygiene-Produkt enthaltend ZnO, wie es in einem der vorhergehenden Ansprüche definiert ist, wobei das Hygiene-Produkt eine Windel für Babys oder für Erwachsene, eine Slip-Einlage oder ein Tampon ist.

8. Verfahren zur Herstellung eines Hygiene-Produkts, das definiert ist wie in Anspruch 7, **dadurch gekennzeichnet, dass** an der Oberfläche chemisch oder physikalisch modifizierte ZnO-Nanopartikel, wie sie in einem der Ansprüche 1 bis 6 definiert sind, auf die Oberfläche des Hygiene-Produkts aufgetragen werden.

9. Das Verfahren nach Anspruch 8, **dadurch gekennzeichnet, dass** das Auftragen des ZnO auf das Hygiene-Produkt durch Tränken (Foulard), Rollenapplikation oder Besprühen des Hygiene-Produkts mit einer Lösung/Suspension der die ZnO-Nanopartikel enthaltenden Avivage und anschließendes Trocknen erfolgt.

## Claims (Claims for the following Contracting State(s): AT, BE, CH, CY, DK, ES, FI, FR, GB, GR, IE, IT, LI, LU, MC, NL, PT, SE, TR)

1. Use of ZnO for the production of hygiene products, the ZnO being present in the form of nanoparticles chemically or physically modified at its surface and modification being intended to mean the coverage of the particle surface with organic compounds which interact with the surface of the particles through chemical bonds or physical forces.

2. Use claimed in claim 1, the hygiene products being selected from diapers for babies and adults, panty liners and tampons.

3. Use claimed in claim 1 or 2, the ZnO having antibacterial (antiseptic) and anti-inflammatory activity.

4. Use claimed in any of the preceding claims, the ZnO being present in the form of nanoparticles chemically or physically modified at its surface which have a mean primary particle size (diameter) of 1 to 100 nm, preferably in the range from 1 to 50 nm or 5 to 40 nm and 10 to 20 nm and more preferably in the range from 5 to 20 nm, 10 to 25 nm or 15 to 35 nm.

5. The use claimed in any of the preceding claims, the ZnO being present in the form of nanoparticles chemically or physically modified at its surface which have a specific surface of at least 10 m²/g, preferably at least 40 m²/g and more preferably at least 100m²/g.

6. Use claimed in any of the preceding claims, the chemical or physical modification of the particle surface being carried out with (a) carboxylic acids (mono-, di- and polycarboxylic acids) or derivatives thereof, such as anhydrides, halides and esters (including lactones); more particularly with stearic acid, palmitic acid, lauric acid, capric acid, caprylic acid, caproic acid, oleic acid, sorbic acid, linoleic acid, linolenic acid, ricinoleic acid, oxalic acid, malonic acid, succinic acid, glutaric acid, adipic acid, citric acid, malic acid, lactic acid, tartaric acid; with (b) amino acids, more particularly with the naturally occurring amino acids (Gly, Ala, Val, Leu, Ile, Phe, Tyr, Trp, Pro, Hy-Pro, Ser, Asp, Glu, Asn, Gln, Arg, Lys, Thr, His, Cys, Met); with (c) hydroxycarboxylic acids and sugar acids, such as glucaric acid, gluconic acid, glucuronic acid; with (d) polyglycolic acids having the general formula HOOC-CH₂-O-(CH₂-CH₂O)ₙ-CH₂-COOH, where n = 1, 2, 3, 4, 5, 6, 7, 8, 9, 10,11 or 12; with (e) ether carboxylic acids having the general formula R-(O-CH₂-CH₂)ₙ-O-CH₂-COOH, where n =1, 2, 3, 4, 5, 6, 7, 8, 9, 10,11 or 12 and R = C₆-, C₈-, C₁₀-, C₁₂-, C₁₄-, C₁₆-, C₁₈-alkyl, alkenyl or alkynyl; with (f) alkyl halides; or with (g) silanes of the type (OR)₄₋ₙSiR'ₙ, where R = methyl, ethyl, propyl, i-propyl, butyl, t-butyl and R' = R = C₆-, C₈-, C₁₀-, C₁₂-, C₁₄-, C₁₆-, C₁₈-alkyl, alkenyl or alkynyl, aminopropyl, N-aminoethyl-3-aminopropyl, n- or i-propyl-N,N,N-dimethyloctadecyl ammonium chloride, n- or i-propyl-N,N,N-trimethyl ammonium chloride, nor i- propylsuccinic anhydride.

7. Use claimed in claim 6, the modification being carried out with stearic acid.

8. Hygiene product containing ZnO as defined in any of the preceding claims.

9. The hygiene product claimed in claim 8 in the form of a diaper for babies or for adults, a panty liner or a tampon.

10. Process for the production of a hygiene product as defined in claim 8 or 9, **characterized in that** ZnO nanoparticles chemically or physically surface-modified as defined in any of claims 1 to 7 are applied to the surface of the hygiene product.

11. The process claimed in claim 10, **characterized in that** the ZnO is applied to the hygiene product by padding (foulard), roller application or spraying of the hygiene product with a solution/suspension of the finish containing the ZnO nanoparticles and subsequent drying.

## Claims (Claims for the following Contracting State(s): DE)

1. Use of ZnO for the production of hygiene products selected from diapers for babies and adults, panty liners and tampons, the ZnO being present in the form of nanoparticles chemically or physically modified at its surface and modification being intended to mean the coverage of the particle surface with organic compounds which interact with the surface of the particles through chemical bonds or physical forces.

2. Use claimed in claim 1, the ZnO having antibacterial (antiseptic) and anti-inflammatory activity.

3. Use claimed in any of the preceding claims, the ZnO being present in the form of nanoparticles chemically or physically modified at its surface which have a mean primary particle size (diameter) of 1 to 100 nm, preferably in the range from 1 to 50 nm or 5 to 40 nm and 10 to 20 nm and more preferably in the range from 5 to 20 nm, 10 to 25 nm or 15 to 35 nm.

4. The use claimed in any of the preceding claims, the ZnO being present in the form of nanoparticles chemically or physically modified at its surface which have a specific surface of at least 10 m²/g, preferably at least 40 m²/g and more preferably at least 100m²/g.

5. Use claimed in any of the preceding claims, the chemical or physical modification of the particle surface being carried out with (a) carboxylic acids (mono-, di- and polycarboxylic acids) or derivatives thereof, such as anhydrides, halides and esters (including lactones); more particularly with stearic acid, palmitic acid, lauric acid, capric acid, caprylic acid, caproic acid, oleic acid, sorbic acid, linoleic acid, linolenic acid, ricinoleic acid, oxalic acid, malonic acid, succinic acid, glutaric acid, adipic acid, citric acid, malic acid, lactic acid, tartaric acid; with (b) amino acids, more particularly with the naturally occurring amino acids (Gly, Ala, Val, Leu, Ile, Phe, Tyr, Trp, Pro, Hy-Pro, Ser, Asp, Glu, Asn, Gln, Arg, Lys, Thr, His, Cys, Met); with (c) hydroxycarboxylic acids and sugar acids, such as glucaric acid, gluconic acid, glucuronic acid; with (d) polyglycolic acids having the general formula HOOC-CH₂-O-(CH₂-CH₂O)ₙ-CH₂-COOH, where n = 1, 2, 3, 4, 5, 6, 7, 8, 9, 10,11 or 12; with (e) ether carboxylic acids having the general formula R-(O-CH₂-CH₂)ₙ-O-CH₂-COOH, where n =1, 2, 3, 4, 5, 6, 7, 8, 9, 10,11 or 12 and R = C₆-, C₈-, C₁₀-, C₁₂-, C₁₄-, C₁₆-, C₁₈-alkyl, alkenyl or alkynyl; with (f) alkyl halides; or with (g) silanes of the type (OR)₄₋ₙSiR'ₙ, where R = methyl, ethyl, propyl, i-propyl, butyl, t-butyl and R' = R = C₆-, C₈-, C₁₀-, C₁₂-, C₁₄-, C₁₆-, C₁₈-alkyl, alkenyl or alkynyl, aminopropyl, N-aminoethyl-3-aminopropyl, n- or i-propyl-N,N,N-dimethyloctadecyl ammonium chloride, n- or i-propyl-N,N,N-trimethyl ammonium chloride, nor i- propylsuccinic anhydride.

6. Use claimed in claim 5, the modification being carried out with stearic acid.

7. Hygiene product containing ZnO as defined in any of the preceding claims, the hygiene product being a diaper for babies or for adults, a panty liner or a tampon.

8. Process for the production of a hygiene product as defined in claim 7, **characterized in that** ZnO nanoparticles chemically or physically surface-modified as defined in any of claims 1 to 6 are applied to the surface of the hygiene product.

9. The process claimed in claim 8, **characterized in that** the ZnO is applied to the hygiene product by padding (foulard), roller application or spraying of the hygiene product with a solution/suspension of the finish containing the ZnO nanoparticles and subsequent drying.

## Revendications (Revendications pour l'(les) Etat(s) contractant(s) suivant(s): AT, BE, CH, CY, DK, ES, FI, FR, GB, GR, IE, IT, LI, LU, MC, NL, PT, SE, TR)

1. Utilisation de ZnO pour la préparation de produits hygiéniques, le ZnO se trouvant sous forme de nanoparticules à surface chimiquement ou physiquement modifiée, la modification étant le recouvrement de la surface des particules par des composés organiques, qui interagissent via des liaisons chimiques ou des forces physiques avec la surface des particules.

2. Utilisation selon la revendication 1, les produits hygiéniques étant choisis parmi les langes pour bébés et adultes, les serviettes hygiéniques et les tampons.

3. Utilisation selon la revendication 1 ou 2, le ZnO ayant un effet antibactérien (antiseptique) et anti-inflammatoire.

4. Utilisation selon l'une quelconque des revendications précédentes, le ZnO se trouvant sous forme de nanoparticules à surface chimiquement ou physiquement modifiée, dont la grosseur moyenne des particules primaires (diamètre) se situe dans la plage de 1 à 100 nm, de préférence dans la plage de 1 - 50 nm ou de 5 - 40 nm et de 10 - 20 nm, de manière particulièrement préférée de 5 à 20 nm, de 10 - 25 nm ou de 15 - 35 nm.

5. Utilisation selon l'une quelconque des revendications précédentes, le ZnO, sous forme de nanoparticules à surface chimiquement ou physiquement modifiée, présentant une surface spécifique d'au moins 10 m²/g, de préférence d'au moins 40 m²/g, et de manière particulièrement préférée d'au moins 100 m²/g.

6. Utilisation selon l'une quelconque des revendications précédentes, la modification chimique ou physique de la surface des nanoparticules étant réalisée avec (a) des acides carboxyliques (acides monocarboxyliques, dicarboxyliques et polycarboxyliques) ou leurs dérivés, tels que les anhydrides, les halogénures et les esters (y compris les lactones) ; en particulier avec l'acide stéarique, l'acide palmitique, l'acide laurique, l'acide caprique, l'acide caprylique, l'acide caproïque, l'acide oléique, l'acide sorbique, l'acide linoléique, l'acide linolénique, l'acide ricinoléique, l'acide oxalique, l'acide malonique, l'acide succinique, l'acide glutarique, l'acide adipique, l'acide citrique, l'acide malique, l'acide lactique, l'acide tartrique; avec (b) des aminoacides, en particulier avec les aminoacides naturels (Gly, Ala, Val, Leu, Ile, Phe, Tyr, Trp, Pro, Hy-Pro, Ser, Asp, Glu, Asn, Gln, Arg, Lys, Thr, His, Cys, Met); avec (c) des acides hydroxycarboxyliques et des acides de sucre, tels que l'acide glucarique, l'acide gluconique, l'acide glucuronique; avec (d) des acides polyglycoliques de formule générale HOOC-CH₂-O-(CH₂-CH₂-O)ₙ-CH₂₋COOH, n représentant 1, 2, 3, 4, 5, 6, 7, 8, 9, 10, 11 ou 12; avec (e) des acides éthercarboxyliques de formule générale R-(O-CH₂-CH₂)ₙ-O-CH₂₋COOH, où n représente 1, 2, 3, 4, 5, 6, 7, 8, 9, 10, 11 ou 12 et où R = un groupe alkyle, alcényle ou alcynyle en C₆, C₈, C₁₀, C₁₂, C₁₄, C₁₆, C₁₈ ; avec (f) des halogénures d'alkyle; ou avec (g) des silanes du type (OR)₄₋ₙSiR'ₙ, où R = méthyle, éthyle, propyle, i-propyle, butyle, t-butyle et R' = un groupe alkyle, alcényle ou alcynyle en C₆, C₈, C₁₀, C₁₂, C₁₄, C₁₆, C₁₈, aminopropyle, N-aminoéthyl-3-aminopropyle, chlorure de n-propyl-N,N,N,-diméthyloctadécylammonium ou chlorure de i-propyl-N,N,N,-diméthyloctadécylammonium, chlorure de n-propyl-N,N,N-triméthylammonium ou chlorure de i-propyl-N,N,N-triméthylammonium, anhydride de l'acide n-propylsuccinique ou i-propylsuccinique.

7. Utilisation selon la revendication 6, la modification étant réalisée avec de l'acide stéarique.

8. Produit hygiénique contenant ZnO, tel qu'il est défini dans une des revendications précédentes.

9. Produit hygiénique selon la revendication 8, le produit hygiénique étant un lange pour bébés ou adultes, une serviette hygiénique ou un tampon.

10. Procédé pour la fabrication d'un produit hygiénique, qui est défini comme dans la revendication 8 ou 9, **caractérisé en ce qu'**on applique des nanoparticules de ZnO à surface chimiquement ou physiquement modifiée, telles que définies dans l'une quelconque des revendications 1 à 7, à la surface du produit hygiénique.

11. Procédé selon la revendication 10, **caractérisé en ce que** l'application du ZnO sur le produit hygiénique est réalisée par imbibition (foulard), application au rouleau ou pulvérisation du produit hygiénique avec une solution/suspension du produit d'avivage contenant les nanoparticules de ZnO et séchage consécutif.

## Revendications (Revendications pour l'(les) Etat(s) contractant(s) suivant(s): DE)

1. Utilisation de ZnO pour la préparation de produits hygiéniques, qui sont choisis parmi les langes pour bébés, et adultes, les serviettes hygiéniques et les tampons, le ZnO se trouvant sous forme de nanoparticules à surface chimiquement ou physiquement modifiée, la modification étant le recouvrement de la surface des particules par des composés organiques, qui interagissent via des liaisons chimiques ou des forces physiques avec la surface des particules.

2. Utilisation selon la revendication 1, le ZnO ayant un effet antibactérien (antiseptique) et anti-inflammatoire.

3. Utilisation selon l'une quelconque des revendications précédentes, le ZnO se trouvant sous forme de nanoparticules à surface chimiquement ou physiquement modifiée, dont la grosseur moyenne des particules primaires (diamètre) se situe dans la plage de 1 à 100 nm, de préférence dans la plage de 1 - 50 nm ou de 5 - 40 nm et de 10 - 20 nm, de manière particulièrement préférée de 5 à 20 nm, de 10 - 25 nm ou de 15 - 35 nm.

4. Utilisation selon l'une quelconque des revendications précédentes, le ZnO, sous forme de nanoparticules à surface chimiquement ou physiquement modifiée, présentant une surface spécifique d'au moins 10 m²/g, de préférence d'au moins 40 m²/g, et de manière particulièrement préférée d'au moins 100 m²/g.

5. Utilisation selon l'une quelconque des revendications précédentes, la modification chimique ou physique de la surface des nanoparticules étant réalisée avec (a) des acides carboxyliques (acides monocarboxyliques, dicarboxyliques et polycarboxyliques) ou leurs dérivés, tels que les anhydrides, les halogénures et les esters (y compris les lactones) ; en particulier avec l'acide stéarique, l'acide palmitique, l'acide laurique, l'acide caprique, l'acide caprylique, l'acide caproïque, l'acide oléique, l'acide sorbique, l'acide linoléique, l'acide linolénique, l'acide ricinoléique, l'acide oxalique, l'acide malonique, l'acide succinique, l'acide glutarique, l'acide adipique, l'acide citrique, l'acide malique, l'acide lactique, l'acide tartrique; avec (b) des aminoacides, en particulier avec les aminoacides naturels (Gly, Ala, Val, Leu, Ile, Phe, Tyr, Trp, Pro, Hy-Pro, Ser, Asp, Glu, Asn, Gln, Arg, Lys, Thr, His, Cys, Met); avec (c) des acides hydroxycarboxyliques et des acides de sucre, tels que l'acide glucarique, l'acide gluconique, l'acide glucuronique; avec (d) des acides polyglycoliques de formule générale HOOC-CH₂-O-(CH₂-CH₂-O)ₙ-CH₂₋COOH, n représentant 1, 2, 3, 4, 5, 6, 7, 8, 9, 10, 11 ou 12; avec (e) des acides éthercarboxyliques de formule générale R-(O-CH₂-CH₂)ₙ-O-CH₂₋COOH, où n représente 1, 2, 3, 4, 5, 6, 7, 8, 9, 10, 11 ou 12 et où R = un groupe alkyle, alcényle ou alcynyle en C₆, C₈, C₁₀, C₁₂, C₁₄, C₁₆, C_{18;} avec (f) des halogénures d'alkyle; ou avec (g) des silanes du type (OR)₄₋ₙSiR'ₙ, où R = méthyle, éthyle, propyle, i-propyle, butyle, t-butyle et R' = un groupe alkyle, alcényle ou alcynyle en C₆, C₈, C₁₀, C₁₂, C₁₄, C₁₆, C₁₈, aminopropyle, N-aminoéthyl-3-aminopropyle, chlorure de n-propyl-N,N,N,-diméthyloctadécylammonium ou chlorure de i-propyl-N,N,N,-diméthyloctadécylammonium, chlorure de n-propyl-N,N,N-triméthylammonium ou chlorure de i-propyl-N,N,N-triméthylammonium, anhydride de l'acide n-propylsuccinique ou i-propylsuccinique.

6. Utilisation selon la revendication 5, la modification étant réalisée avec de l'acide stéarique.

7. Produit hygiénique contenant ZnO, tel qu'il est défini dans une des revendications précédentes, le produit hygiénique étant un lange pour bébés ou adultes, une serviette hygiénique ou un tampon.

8. Procédé pour la fabrication d'un produit hygiénique, qui est défini comme dans la revendication 7, **caractérisé en ce qu'**on applique des nanoparticules de ZnO à surface chimiquement ou physiquement modifiée, telles que définies dans l'une quelconque des revendications 1 à 6, à la surface du produit hygiénique.

9. Procédé selon la revendication 8, **caractérisé en ce que** l'application du ZnO sur le produit hygiénique est réalisée par imbibition (foulard), application au rouleau ou pulvérisation du produit hygiénique avec. une solution/suspension du produit d'avivage contenant les nanoparticules de ZnO et séchage consécutif.
